(19) Europäisches Patentamt
European Patent Office
Office européen des brevets

(11) Publication number: **0 122 654**
**B1**

(12) # EUROPEAN PATENT SPECIFICATION

(45) Date of publication of patent specification: **10.12.86**

(51) Int. Cl.⁴: **C 07 C 149/06**, C 07 C 148/00

(21) Application number: **84200420.2**

(22) Date of filing: **23.03.84**

(54) **Preparation of secondary thiols.**

(30) Priority: **11.04.83 US 484051**

(43) Date of publication of application:
**24.10.84 Bulletin 84/43**

(45) Publication of the grant of the patent:
**10.12.86 Bulletin 86/50**

(84) Designated Contracting States:
**BE DE FR GB IT NL**

(56) References cited:
**US-A-4 102 931**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Fried, Herbert Elliott**
**10615 Briar Forest Drive No. 404**
**Houston Texas (US)**

(74) Representative: **Aalbers, Onno et al**
**P.O. Box 302**
**NL-2501 CH The Hague (NL)**

**EP 0 122 654 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to the preparation of $C_{10}$ to $C_{30}$ secondary thiols by reaction of hydrogen sulphide with linear mono-olefins.

Thiols in the $C_{10}$ to $C_{30}$ range are known compounds. Secondary thiols have utility in applications as odorants, components of lubricant formulations, and curing agents for epoxy resins, but are further of particular advantage when used as intermediates in the synthesis of surfactant chemicals.

It is known that thiols (mercaptans) can be prepared in a process which comprises the addition of hydrogen sulphide to olefins, particularly in the presence of a catalyst, most particularly an acid catalyst. It is further recognized that this process has been applied almost exclusively to the production of tertiary thiols. Markovnikov addition of $H_2S$ to a "tertiary" olefin, usually an olefin polymer such as a propylene or butylene trimer or tetramer, results in near quantitative selectivity to the tertiary thiol.

The preparation of secondary thiols from higher olefins in a similar manner has not been a practical success. In one regard, the preparation of secondary thiols from linear olefins by conventional methods for $H_2S$ addition has been accompanied by the formation of substantial quantities of dialkyl sulphide by-product. The thiol is the addition product of one $H_2S$ molecule and one olefin molecule. Dialkyl sulphide is formed when the thiol, once produced, reacts with an additional molecule of the olefin. In known processes for preparing secondary thiols from $H_2S$ and higher olefins, dialkyl sulphide is formed in a quantity between about 50 and 100 percent by weight, calculated on secondary thiol. (For primarily steric reasons, dialkyl sulphide production is not a significant problem in the preparation of tertiary thiols from branched olefins). In another regard, the catalysts and reaction conditions which are applied to promote the addition of $H_2S$ to olefins also promote double bond isomerization and/or rearrangement of the molecular structure of the olefin. As a result, olefins which might be expected to be converted to secondary thiols upon $H_2S$ addition are instead converted to tertiary thiols.

The preparation of secondary thiols, particularly secondary thiols of linear carbon chain structure, is most important, if the thiols are to be suitable for use as intermediates in the synthesis of surface active agents. Among the surfactants which can be derived from thiols in the $C_{10}$ to $C_{30}$ range are the anionic paraffin sulphonates (molecules of the general formula $R—SO_3^-M^+$, where R is $C_{10}$ to $C_{30}$ alkyl and M is a cation such as sodium) which are prepared, for example, by oxidizing the thiol. Secondary thiols are particularly useful in the preparation of nonionic thiol alkoxylates (of the general formula $R—S{+}R'—O{+}_xH$, where R is again $C_{10}$ to $C_{30}$ alkyl, R′ is $C_2$, $C_3$, or $C_4$ alkyl, and x is an integer between about 1 and 30), which may be prepared by the contact of the thiol with a $C_2$ to $C_4$ alkylene oxide at elevated temperature (e.g., 140°C) and pressure (e.g., 7 bar) in the presence of an acidic (Lewis acid) or a basic (alkaline or alkaline earth metal) catalyst.

One factor of obvious importance to the use of surfactants in detergent service is their capabilities for soil removal. Surfactants produced from secondary thiols have been found to have excellent cleaning properties in a wide variety of detergent applications. Another important factor in surfactant utilization has to do with environmental considerations. In many of their common applications in both industry and the home, surfactants find their way into waste water streams. Biodegradation of the surfactant molecule then becomes of critical concern. Branched carbon chain surfactants derived from tertiary thiols are significantly less biodegradable than those surfactant molecules of linear carbon chain that are derived from secondary thiols, and, accordingly, are much less acceptable for widespread use in detergent and other common surfactant services. For this reason, an improved process for the selective preparation of secondary thiols would be particularly desirable.

With specific regard to catalysts utilized in the process of the present invention, U.S. Patent Specification 4,102,931 describes the use of zeolites to catalyze the addition of $H_2S$ to branched unsymmetrical olefins for preparation of tertiary thiols. Linear olefins are excluded from the starting material disclosed as useful in this prior art process, and the patent does not attribute to the zeolites any beneficial influence upon any aspect of process selectivity.

It has now been found that secondary thiols are prepared in high selectivity by the addition of $H_2S$ to $C_{10}$ to $C_{30}$ linear mono-olefins in the presence of certain zeolite catalysts.

Accordingly, the present invention relates to a process for the preparation of secondary thiols of the structure

$$R^1—CH—R^2$$
$$SH$$

wherein $R^1$ and $R^2$ are each alkyl and $R^1$ and $R^2$ together with the carbon atom to which the —SH group is attached, form a linear chain of from 10 to 30 carbon atoms which comprises contacting in a liquid phase at a temperature in the range from 40° to 140°C and at a pressure of at least 10 bar, one or more $C_{10}$ to $C_{30}$ linear mono-olefins with hydrogen sulphide, the molar ratio of said hydrogen sulphide to said olefins in said liquid phase being at least 1 to 1, in the presence of a catalytically effective amount of a zeolite catalyst.

Of particular importance, this process results in production of secondary thiols in high selectivity. Problems of undesirable conversion of the olefin starting material to dialkyl sulphide and/or tertiary thiol

by-products, characteristic of all prior art processes for preparation of secondary thiols in the higher carbon number range, have been essentially eliminated.

The process of the invention is intended for limited application to the preparation of secondary thiols in the carbon number range from about 10 to 30 from corresponding $C_{10}$ to $C_{30}$ mono-olefins. Preference in this regard may be expressed for application of the invention to the conversion of olefins in the carbon number range from about 10 to 22, while an olefin reactant in the carbon number range from about 10 to 16 is more preferred, and an olefin in the carbon number range from about 10 to 14 is considered most preferred. The invention is further intended for the preparation of secondary thiols from olefins of linear (straight chain) structure. Similar processing of branched or cyclic olefins leads to the formation of significant quantities of materials other than the secondary thiols.

Preferred for use as olefin reactant for the practical reason of availability are the commercial olefin products in the $C_{10}$ to $C_{30}$ range. One example of such olefins is the Chevron Alpha Olefin product series (trademark of and sold by Chevron Chemical Co.), manufactured by the cracking of paraffin wax. Commercial production is more commonly accomplished by the oligomerization of ethylene using procedures well known to the art. The resulting oligomerization products are substantially of linear structure and thus products are substantially of linear structure and thus readily distinguishable from the highly branched propylene and butylene oligomers (or polymers) conventionally used to prepare tertiary thiols. Commercial olefin products manufactured by ethylene oligomerization are marketed in the United States by Gulf Oil Chemicals Company under the trademark Gulfene, by Shell Chemical Company under the trademark Neodene and by Ethyl Corporation as Ethyl Alpha-Olefins. While most of such olefin products are comprised largely of alpha-olefins, higher linear internal olefins are also commercially produced, for example, by the chlorination-dehydrochlorination of paraffins, by paraffin dehydrogenation, and by isomerization of alpha-olefins. An olefin starting material containing a major proportion of internal olefins is particularly preferred as starting material in the process of the invention, from the standpoint of both reaction rate and selectivity to secondary thiol. Linear internal olefin products in the $C_{10}$ to $C_{30}$ range are marketed by Shell Chemical Company and by Liquichemica Company. These commercial products, whether predominantly internal or alpha-olefins typically contain about 70 percent by weight (%w) or more, most often about 80 %w or more, linear mono-olefins in a specified carbon number range (e.g., $C_{10}$ to $C_{12}$, $C_{11}$ to $C_{15}$, $C_{12}$ to $C_{13}$, $C_{15}$ to $C_{18}$, etc.), the remainder of the product being olefin of other carbon number or carbon structure, diolefins, paraffins, aromatics, and other impurities resulting from the synthesis process. Commercial olefins consisting essentially of (i.e., containing at least about 90 %w) linear mono-olefins in the specified carbon number range are considered most preferred for use as reactant in the process of the invention.

The $H_2S$ reactant is suitably obtained from any convenient source, although it is preferably in a relatively pure form. It is particularly important that the $H_2S$ reactant and the reaction system as a whole be essentially free of water, the presence of which is found to result in loss of catalyst activity. Lewis bases and organic peroxides should also be eliminated from the reaction system.

For purposes of this invention, the olefin and $H_2S$ reactants are contacted in the presence of a catalytically effective amount of one or more of certain zeolites. As the terminology is understood in the art and as it is used herein, the zeolites are a family of crystalline aluminosilicates well defined both as to chemical composition and physical structure. Chemically, the zeolites may be represented by the formula

$$M_{x/n}[(AlO_2)_x \,.\, (SiO_2)_y]mH_2O,$$

wherein M is a cation of valence n. Structurally, $AlO_4$ and $SiO_4$ groups are linked by shared oxygen atoms in a three dimensional network forming intracrystal cavities interconnected by smaller channels, or pores. Cations and water molecules are bound to the aluminosilicate framework within the cavities. The various zeolites differ one from the other principally in terms of the proportion of Al to Si, the identity of the cation, the configuration of the three dimensional alumina and silica network, and the particular size and shape of the crystal pores and cavities.

Two aspects of the zeolites, one relating to structure and the other to composition, have been found to be critical to their successful application as the catalyst in the process of the invention. In terms of structure, the pore openings of a suitable zeolite necessarily have a diameter of at least 0.4 nm, and are preferably of a diameter no greater than 0.9 nm. Particularly preferred is a zeolite having pore openings between 0.5 nm and 0.9 nm, while a zeolite with a pore diameter in the range from 0.5 nm to 0.85 nm is considered to be optimal. Type X and Type Y zeolites, and zeolites of the ZSM series are specific examples of synthetic zeolites having pore openings in this most preferred range. Also suitable for use in the invention are natural (or mineral) zeolites having the specified pore opening, such as mordenite, for example. In terms of composition, the zeolite is necessarily of relatively high acidity, a requirement which relates to the nature of the cation present in the alumina and silica network. The monovalent alkali metal cation (e.g. $Na^+$ or $K^+$) form in which the synthetic zeolites are commonly found or produced does not possess the necessary acidity. However, exchange can be carried out, under procedures well known in the art, to replace the alkali metals with certain other cations and to thereby impart to the zeolite activity and the desired high selectivity for thiol preparation when applied according to the invention. Particularly suitable for purposes of the invention are decationized, or protonated, zeolites and zeolites having one or more cations selected

from the group consisting of magnesium and the rare earth metals (elements of atomic numbers 21, 39 and 57 to 71, inclusive). Cation exchange can be accomplished simply by contacting the zeolite at elevated temperature (e.g., 100°C) with a series of aqueous solutions containing the desired replacement cation. Decationized zeolites may be prepared by an exchange with an ammonium ion, followed by heating to a temperature of about 550°C. Cation exchange procedures typically result in replacement of between about 70 and 95% of the zeolite cations.

As examples of specific synthetic zeolites preferred for use in this invention mention may be made of commercial product marketed by Union Carbide Corporation, particularly the decationized Y zeolites known as Linde LZ-Y62, LZ-Y72, and LZ-Y82, and the rare earth impregnated Y zeolite Linde SK-500. Other examples are the ZSM zeolites produced by Mobil Corporation (e.g., ZSM-4, ZSM-5, ZSM-11, ZSM-12, and ZSM-38) the preparation and properties of which are illustrated, for instance, by U.S. patent specifications 3,702,886 and 4,046,859. Examples of preferred natural or mineral zeolites include cation-exchanged or decationized mordenite, ferrierite, gmelinite, cancrinite heulandite, and dachiardite. Mordenite is particularly preferred.

Prior to use, the catalyst is preferably calcined, for instance, by heating to a temperature between about 250° and 750°C for several hours to remove adsorbed water. A temperature of about 500°C has been found to be particularly useful. Calcination may not be necessary for a freshly obtained zeolite, but should be carried out if the catalyst has been exposed to air or water.

For practice in accordance with the invention, $H_2S$ and olefin are contacted in the liquid phase with the solid catalyst under necessarily restricted conditions of temperature, pressure, and relative proportions of catalyst and reactants.

Contact between the olefin and $H_2S$ takes place in the liquid phase. For purposes of achieving high selectivity to the secondary thiol, it is critical that this liquid phase contains $H_2S$ and olefin in a molar ratio of at least 1 to 1. Depending upon temperature and pressure, the reaction zone may also contain an $H_2S$-rich vapour phase, although only the $H_2S$ present in the liquid phase is included in calculating the necessary molar ratio relative to olefin. Higher selectivity to secondary thiol is generally realized with increases in the $H_2S$ to olefin molar ratio above 1 to 1. For this reason, an $H_2S$ to olefin ratio in the liquid phase of at least 2 to 1 by mole is preferred, a ratio of at least 3 to 1 by mole is more preferred, and a ratio of at least 5 to 1 by mole, particularly at least 10 to 1, is considered most preferred.

The process is suitably carried out only within a limited range of temperatures. To some extent, suitable process temperature is dependent upon the particular nature of the olefin reactant. In the case of a reactant which is comprised substantially of internal olefin, a temperature in the range from 40° to 140°C is very suitable, and a temperature in the range from 50° to 120°C, particularly from 60° to 100°C, is preferred. For a reactant comprised substantially of alpha-olefin, the process can be conducted in the same 40° to 140°C range, although desired selectivity is then realized only at somewhat higher ratios of $H_2S$ to olefin, i.e., ratios greater than 1.5 to 1 by mole, particularly greater than 5 to 1 by mole. Preference is given to the processing of alpha-olefins at a temperature from 50° to 100°C, particularly to a temperature from 60° to 85°C. In each case, the $H_2S$ addition reaction does not proceed at appreciable rate at temperatures below 40°C, while at temperatures above 140°C the process does not realize the desired high selectivity to secondary thiol at desirable process pressures.

The invention is necessarily carried out under pressure, preferably at a pressure greater than 17 bar, although somewhat lower pressures (at least 10 bar) may be suitable at the lower process temperatures. Attention to such limitations on process pressure is critical to the successful practice of the invention. An increase in pressure, at a given temperature, is beneficial to the selective production of the secondary thiols, since it provides opportunity for maintaining a higher $H_2S$ to olefin molar ratio in the liquid phase. Considered to be particularly preferred from the standpoint of both process selectivity and processing convenience is a pressure of at least 25 bar, and particularly from 40 to 100 bar. Preferences expressed with regard to upper limits on process pressure relate to aspects of practical equipment design rather than to process performance, and significantly higher pressures can be applied if desired.

The olefin and $H_2S$ are contacted in the presence of a quantity of the catalyst which is effective for promoting the desired conversion to thiol. In quantitative terms, and using a batch process and a powered catalyst as an example, a catalytically effective amount of the zeolite is ordinarily in excess of 3 percent by weight (%w), calculated on the weight of the olefin reactant. Larger amounts of the catalyst, e.g., greater than 6%w are usually preferred from the standpoint of enhanced reaction rate and selectivity to secondary thiol, while still larger amounts, e.g., greater than 10 %w, particularly greater than 20 %w, are more preferred. As a general rule, larger quantities will be necessary if the catalyst is applied in pellet or extrudate, rather than powdered form.

As a specific example of procedures which can be applied in the practice of the invention, liquid $H_2S$ and liquid olefin in suitable relative proportions are continuously mixed at low temperature, heated to the desired process temperature and passed through one or more contained beds of the catalyst. The process is equally adaptable to a batch mode of operation, for instance, one in which the liquid $H_2S$ and olefin mixture is added to a reaction zone containing a suitable quantity of catalyst and maintained at the desired process temperature with agitation. Under preferred conditions of temperature and pressure and relative proportions of reactants and catalyst, essentially complete conversion of olefin to thiol is typically achieved in 2 to 15 hours, often in 3 to 7 hours.

Thiol product is suitably recovered from the process product mixture by generally conventional methods. For example, $H_2S$ reactant is effectively flash evaporated from the liquid thiol at near atmospheric pressure and at a temperature of about 125°C. Stripping with an inert gas, such as nitrogen, promotes $H_2S$ removal. The remaining liquid may be vacuum distilled to separate thiol product from the typically higher boiling by-product compounds, particularly the dialkyl sulphides.

The product of the process of the invention is a secondary thiol of the formula

$$R^1-\underset{\displaystyle |}{\overset{SH}{CH}}-R^2,$$

wherein $R^1$ and $R^2$ are each alkyl, $R^1$ and $R^2$, together with the carbon atom to which the SH moiety is attached, form a linear chain of between 7 and 30 carbon atoms. Position of the SH substitution along this chain is to some extent dependent upon the position of the double bond in the olefin starting material. However, it is observed that during practice of the invention isomerization of the olefin, with respect to double bond position, occurs at a rate that is roughly equivalent to the rate of $H_2S$ addition, producing a product in which the position of SH substitution is largely random. Such double bond isomerization necessitates the use of a linear olefin reactant to insure selective production of secondary rather than tertiary thiols. Moreover, the use of the linear olefin in the process of the invention is found to be sufficient to result in production of essentially only the secondary thiol. Although the starting material is subject to double bond isomerization, it is not found to undergo any significant degree of rearrangement in the carbon structure to form a tertiary olefin which would in turn yield the tertiary rather than the secondary thiol.

The invention is further illustrated by the following Examples, representing practice under certain preferred embodiments of the process of the invention.

Examples 1—12

A series of examples of the process of the invention was carried out in a batch mode. For each experiment, measured amounts of an olefin starting material and a rare earth impregnated Y-zeolite (Linde SK-500) were introduced into an autoclave reactor. Several olefin reactants, differing in carbon number and double bond position, were used. The catalyst was in the form of an extrudate, and contained 10.7 %w mixed rare earth oxides. To eliminate variations in activity caused by exposure to water or air the catalyst was calcined before use (i.e., at a temperature of 500°C for about 12 hours). Precautions were also taken in loading the olefin and catalyst to maintain the autoclave and its contents essentially oxygen and water-free. Following introduction of catalyst and olefin, the autoclave was sealed, purged with an inert gas, and then cooled to −70°C. $H_2S$ reactant, in liquid form, was added at this temperature. Sufficient $H_2S$ was added to give a molar ratio of total $H_2S$ in the autoclave to total olefin in the autoclave of at least 3.3. In each case the molar ratio of $H_2S$ to olefin in the liquid phase in the autoclave was calculated to be greater than 1. The autoclave was then heated to the desired process temperature and autoclave pressure (maximum process pressure) was determined. (In each case, this pressure was in the range from about 25 to 60 bar). Essentially constant temperature was maintained for a desired time by controlled cooling of the reactor. Analysis of the resulting liquid reaction mixture was conducted, after flashing or stripping off excess $H_2S$ reactant. Conversion of olefin starting material was determined by gas-liquid chromatography. Selectivity to secondary thiol was determined either by gas-liquid chromatography or by titration with silver nitrate. Dialkyl sulphide by-product was determined by HPLC analysis. Results of Examples 1—12 are presented in Table I.

TABLE I

| Example No. | $H_2S$ to olefin* molar ratio | Catalyst (weight percent on olefin) | Temp. (°C) |
|---|---|---|---|
| 1[a] | 8.33 | 29.33 | 100 |
| 2[b] | 8.25 | 33.3 | 60 |
| 3[c] | 17.18 | 33.3 | 60 |
| 4[a] | 8.33 | 29.8 | 60 |
| 5[a] | 8.88 | 29.8 | 60 |
| 6[d] | 8.33 | 29.8 | 60 |
| 7[b] | 8.25 | 33.3 | 60 |
| 8[b] | 8.25 | 33.3 | 60 |
| 9[e] | 8.25 | 33.3 | 60 |
| 10[e] | 8.25 | 33.3 | 60 |
| 11[e] | 3.30 | 33.3 | 60 |
| 12[e] | 3.30 | 33.3 | 60 |
| 13[a] | 50 | 33.3 | 100 |

* calculated on the basis of the total weight of $H_2S$ and the total weight of olefin added to the autoclave.
[a] $C_{16}$ internal olefin reactant
[b] $C_{12}$ internal olefin reactant
[c] $C_{14}$ internal olefin reactant
[d] $C_{16}$ alpha-olefin reactant
[e] $C_{12}$ alpha-olefin reactant.

TABLE (continued)

| Example No. | Reaction time (h) | Olefin conversion | Selectivity to secondary thiol |
|---|---|---|---|
| 1 | 4 | 99 | 82 |
| 2 | 4 | 99 | 95 |
| 3 | 4 | 99 | 90 |
| 4 | 24 | 99 | 95 |
| 5 | 5 | 99 | 91 |
| 6 | 23 | 93 | 86 |
| 7 | 17 | 97 | 96 |
| 8 | 4 | 99.9 | 95 |
| 9 | 4 | 95 | 90 |
| 10 | 4 | 87 | 90 |
| 11 | 72 | 99 | 84 |
| 12 | 72 | 99 | 91 |
| 13 | 2.5 | 95 | 95 |

Example 14

A continuous process in accordance with the invention was carried out by passing a mixture of $C_{11}/C_{12}$ internal olefin and liquid $H_2S$, at a temperature of about 60°C and a pressure of about 40 bar, through a contained bed of a decationized Y zeolite (Linde LZ-Y82). The molar ratio of $H_2S$ to olefin was about 20 to 1 in the feed (all liquid phase) entering the bed. Flowrate of reactants was controlled to give a liquid hourly space velocity of about 1.0, calculated on olefin. Under these conditions, conversion of olefin was essentially complete, with a selectivity to secondary thiol of 98%.

**Claims**

1. A process for the preparation of secondary thiols of the structure

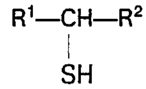

wherein $R^1$ and $R^2$ are each alkyl and $R^1$ and $R^2$ together with the carbon atom to which the —SH group is attached, form a linear chain of from 10 to 30 carbon atoms which comprises contacting in a liquid phase at a temperature in the range from 40° to 140°C and at a pressure of at least 10 bar, one or more $C_{10}$ to $C_{30}$ linear mono-olefins with hydrogen sulphide, the molar ratio of said hydrogen sulphide to said olefins in said liquid phase being at least 1 to 1, in the presence of a catalytically effective amount of a zeolite catalyst.

2. A process as claimed in claim 1, wherein the olefins have a carbon number in the range from 10 to 22.

3. A process as claimed in claim 1 or 2, wherein the molar ratio of hydrogen sulphide to olefins in the liquid phase is at least 3 to 1.

4. A process as claimed in claims 1—3, wherein the pressure is at least 25 bar and the temperature is in the range from 50° to 100°C.

5. A process as claimed in claims 1—4, wherein the zeolite is a decationized or cation exchanged zeolite having pore openings between 0.4 and 0.9 nm.

6. A process as claimed in claim 5, wherein the cation-exchanged zeolite is one in which the replacement cation is magnesium or a rare earth metal.

7. A process as claimed in claim 1, 5 or 6, wherein the zeolite is Type X, Type Y, mordenite, or a ZSM zeolite.

8. A process as claimed in claim 7, wherein the zeolite has pore openings between 0.5 and 0.85 nm.

9. A process as claimed in claims 1—8, wherein the molar ratio of hydrogen sulphide to olefins in the liquid phase is at least 5 to 1, the temperature is in the range from 60° to 85°C, and the pressure is in the range from 40 to 100 bar.

**Patentansprüche**

1. Ein Verfahren zur Herstellung von sekundären Thiolen mit der Struktur

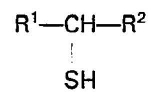

in welcher $R^1$ und $R^2$ jeweils Alkyl darstellt und $R^1$ und $R^2$ zusammen mit dem Kohlenstoffatom, an welches die —SH-Gruppe gebunden ist, eine lineare Kette mit 10 bis 30 Kohlenstoffatomen bilden, welches Verfahren das Kontaktieren von 1 oder mehreren linearen $C_{10}$—$C_{30}$-Monoolefinen mit Schwefelwasserstoff in einer flüssigen Phase bei einer Temperatur im Bereich von 40 bis 140°C und bei einem Druck von mindestens 10 Bar in Anwesenheit einer katalytisch wirksamen Menge eines Zeolith-Katalysators umfaßt, wobei das Molverhältnis von besagtem Schwefelwasserstoff zu besagten Olefinen in besagter flüssiger Phase mindestens 1:1 beträgt.

2. Ein Verfahren wie in Anspruch 1 beansprucht, in welchem die Olefine eine Kohlenstoffzahl im Bereich von 10 bis 22 aufweisen.

3. Ein Verfahren wie in Anspruch 1 oder 2 beansprucht, in welchem das Molverhältnis von Schwefelwasserstoff zu den Olefinen in der flüssigen Phase mindestens 3:1 beträgt.

4. Ein Verfahren wie in den Ansprüchen 1 bis 3 beansprucht, in welchem der Druck mindestens 25 Bar beträgt und die Temperatur im Bereich von 50 bis 100°C liegt.

5. Ein Verfahren wie in den Ansprüchen 1 bis 4 beansprucht, in welchem der Zeolith ein entkationisierter oder ein kationausgetauschter Zeolith mit Porenöffnungen zwischen 0,4 und 0,9 nm ist.

6. Ein Verfahren wie in Anspruch 5 beansprucht, in welchem der kationausgetauschte Zeolith ein solcher ist, in welchem das durch Austausch eingebrachte Kation Magnesium oder ein seltenes Erdmetall ist.

7. Ein Verfahren wie in Anspruch 1, 5 oder 6 beansprucht, in welchem der Zeolith ein solcher des Typs X, des Typs Y, Mordenit oder ein ZSM-Zeolith ist.

8. Ein Verfahren wie in Anspruch 7 beansprucht, in welchem der Zeolith Porenöffnungen zwischen 0,5 und 0,85 nm aufweist.

9. Ein Verfahren wie in den Ansprüchen 1 bis 8 beansprucht, in welchem das Molverhältnis von Schwefelwasserstoff zu den Olefinen in der flüssigen Phase mindestens 5:1 beträgt, die Temperatur im Bereich von 60 bis 85°C und der Druck im Bereich von 40 bis 100 Bar liegt.

**Revendications**

1. Procédé de préparation de thiols secondaires de structure

$$R^1—CH—R^2$$
$$|$$
$$SH$$

où R1 et R2 représentent chacun un alcoyle et R1 et R2 ensemble avec l'atome de carbone auquel le groupe —SH est attaché, forment une chaîne linéaire en C10 à C30 dans lequel on met en contact en phase liquide à une température située dans un intervalle allant de 40° à 140°C et à une pression d'au moins 10 bars, une ou plusieurs mono-oléfines linéaires en C10 à C30 avec du sulfure d'hydrogène, le rapport molaire dudit sulfure d'hydrogène auxdites oléfines dans ladite phase liquide étant d'au moins 1 à 1, en présence d'une quantité catalytiquement efficace d'un catalyseur zéolithe.

2. Procédé selon la revendication 1, dans lequel les oléfines ont un nombre de carbones situé dans l'intervalle allant de 10 à 22.

3. Procédé selon les revendications 1 et 2, dans lequel le rapport molaire du sulfure d'hydrogène aux oléfines dans la phase liquide est d'au moins 3 à 1.

4. Procédé selon l'une des revendications 1 à 3, dans lequel la pression est d'au moins 25 bars et la température est dans un intervalle allant de 50° à 100°C.

5. Procédé selon l'une des revendications 1 à 4, dans lequel la zéolithe est une zéolithe ayant subi une décationisation ou un échange de cations et ayant des ouvertures de pores comprises entre 0,4 et 0,9 nm.

6. Procédé selon la revendication 5, dans lequel la zéolithe ayant subi un échange de cations est une zéolithe dans laquelle le cation de remplacement est le magnésium ou un métal de terre rare.

7. Procédé selon les revendications 1, 5 et 6, dans lequel la zéolithe est de type X, de type Y, la mordénite ou une zéolithe ZSM.

8. Procédé selon la revendication 7, dans lequel la zéolithe a des ouvertures de pores comprises entre 0,5 et 0,85 nm.

9. Procédé selon les revendications 1—8, dans lequel le rapport molaire du sulfure d'hydrogène aux oléfines dans la phase liquide est d'au moins 5 à 1, la température est dans l'intervalle allant de 60° à 85°C, et la pression est dans l'intervalle allant de 40 à 100 bars.